# NEUE EUROPÄISCHE PATENTSCHRIFT

(11) **EP 0 460 190 B2**
(45) Veröffentlichungstag und Bekanntmachung des Hinweises auf die Entscheidung über den Einspruch: **28.11.2001**
(45) Hinweis auf die Patenterteilung: 29.03.1995
(21) Anmeldenummer: 91902191.5
(22) Anmeldetag: 27.12.1990
(51) Int. Cl.: G01N 33/574, G01N 33/547, C12P 21/08, C07K 16/00

(54) **VERFAHREN ZUM NACHWEIS VON MALIGNEN ERKRANKUNGEN**
METHOD FOR DETECTING MALIGNANT DISEASES
PROCEDE DE DEPISTAGE DE MALADIES MALIGNES

(30) Priorität: 27.12.1989 DE 3942999
(43) Veröffentlichungstag der Anmeldung: 11.12.1991
(73) Patentinhaber: Roche Diagnostics GmbH, 68298 Mannheim (DE)
(72) Erfinder: BODENMÜLLER, Heinz, D-8132 Tutzing (DE); DESSAUER, Andreas, D-8132 Tutzing (DE)
(74) Vertreter: Schüssler, Andrea, Dr.
(86) Internationale Anmeldenummer: EP9002314
(87) Internationale Veröffentlichungsnummer: WO9110139

(56) Entgegenhaltungen:
- EP-A- 0 269 092
- EP-A- 0 337 057
- WO-A-85/03132
- LABORATORY INVESTIGATION, vol. 55, no. 4, 1986, The US-Canadian Division of the Internatl. Academy of Pathology, US; M. OSBORN et al., pp. 497-504#
- JOURNAL OF INVESTIGATIVE DERMATOLOGY, vol. 92, no. 5, May 1989, Society for Investigative Dermatology Inc.; P.C. STASIAK et al., pp. 707-716#
- The International Journal of Biological Markers, 9(2), 1994,75-81
- Gastroenterology, 96(5 part 2), A673, May 1989
- Achstätter T., Doctoral Thesis, University of Heidelberg, 1988
- Stasiak P C, Doctoral Thesis, University of London, 1988

## Beschreibung

Die Erfindung betrifft ein Verfahren zum Nachweis von malignen Erkrankungen und ein dazu geeignetes Reagenz.

In der klinischen Diagnostik wird schon sehr lange nach einer Indikatorsubstanz gesucht, die einen Hinweis auf maligne Erkrankungen gibt. Als Tumormarker mit breiter Organspezifität wurden Hoffnungen gesetzt auf CEA (Carcinoembrionales Antigen) und TPA (Tissue Polypeptide Antigen). Inzwischen stellte sich jedoch heraus, daß beide Proteine diese Erwartungen nicht erfüllen konnten. Der Einsatz von CEA hat sich mittlerweile im wesentlichen auf die Therapieüberwachung z.B. von kolorektalen Karzinomen reduziert, während sich TPA wegen mangelnder Spezifität und Sensitivität nur für wenige Indikationen zur Therapieüberwachung durchsetzen konnte. Sehr viele andere Proteine wurden bereits als Indikatoren für maligne Erkrankungen getestet, konnten jedoch alle nicht befriedigen.

Mit Tumoren in Verbindung gebracht wurde auch die Klasse der Cytokeratine (CK). Diese sind als Intermediär-Filamentproteine Bestandteile des Zytoskelettes von Epithelzellen, Es sind 19 Cytokeratine bekannt, von denen die Cytokeratine 1 bis 8 als basische und die Cytokeratine 9 bis 19 als saure Cytokeratine bezeichnet werden. Die Cytokeratine können sich in der Zelle zu Tetrameren zusammenlagern. Ein Tetramer besteht dabei aus jeweils zwei basischen und zwei sauren Cytokeratin-Molekülen. Durch lineare Aggregation von Tetrameren entstehen Filamente. Intakte Cytokeratin-Moleküle sind als integrale Bestandteile der Intermediär-Filamente epithelialer Zellen wasserunlöslich. Die Komplexität und Zusammensetzung der Cytokeratine ist unterschiedlich in den verschiedenen epithelialen Geweben, d.h. Epithelzellen haben für das Gewebe typische Cytokeratin-Zusammensetzungen. Es ist bisher allerdings nichts darüber bekannt, daß maligne Erkrankungen mit dem Auftreten von Cytokeratinen in Körperflüssigkeiten korrelieren.

Es war nun Aufgabe dervorliegenden Erfindung, ein Verfahren zur Verfügung zu stellen, mit dem das Vorliegen einer malignen Erkrankung diagnostiziert werden kann.

Diese Aufgabe wird gelöst durch ein Verfahren zum Nachweis von malignen Erkrankungen, das dadurch gekennzeichnet ist, daß man die Probe einer Körperflüssigkeit mit mindestens zwei Rezeptoren R₁ und R₂ inkubiert, wobei durch Bindung mindestens der Rezeptoren R₁ und R₂ mit der in der Probelösung nachzuweisenden Substanz eine Signaländerung erzeugt wird und wobei einer der beiden Rezeptoren monoklonalen Antikörper enthält, welcher an die Aminosäuresequenz 311 bis 335 von Cytokeratin 19 bindet und der andere Rezeptor einen monoklonalen Antikörper, welcher an die Aminosäuresequenz 346 bis 367 von Cytokeratin 19 bindet, enthält und die durch die Bindung verursachte Signaländerung in der Probe bestimmt.

Überraschenderweise wurde festgestellt, daß vorwiegend in epithelialen Tumorgeweben Fragmente von Cytokeratin 19 entstehen, welche die Sequenz 311 bis 359 des kompletten Cytokeratin 19 besitzen und die Epitope auf den Äminosäuresequenzen 291 bis 335 und 346 bis 367 aufweisen, die mit den oben angegebenen monoklonalen Antikörpern oder deren Derivaten spezifisch binden können und zudem in Körperflüssigkeiten zu finden sind. Cytokeratin 19 (CK 19) besitzt eine Aminosäurekette aus 400 Aminosäuren. Die Sequenz ist beschrieben in Stasiak, P.C. et al., J.Invest.Dermatol. 92 (1989) 707-716, besonders Seite 712 und ist in 3 Domänen 1A, 1B und 2 gegliedert. Die erfindungsgemäß nachzuweisenden Fragmente stammen aus der Domäne 2 (Helix 2). Es wurde gefunden, daß diese CK 19-Fragmente, die mit den beiden obengenannten Antikörpern spezifisch binden, insbesondere bei Bronchial-, Mamma-, Magen-, Gallenweg-, Leberund Kolonkarzinomen nachweisbar sind. Bei Patienten mit entzündlichen epithelialen Krankheiten der entsprechenden Gewebe konnten diese Fragmente in der Regel nicht nachgewiesen werden.

Bevorzugt werden für die Erfindung die monoklonalen Antikörper verwendet, welche von den Zellinien ECACC 89112803 (bindet an Sequenz 291 bis 335, vorzugsweise an die Sequenz 311 bis 335) und ECACC 89112804 (bindet an Sequenz der Aminosäuren 346 bis 367, vorzugsweise an 346 bis 359) gebildet werden.

Andere mit CK 19 bindende monoklonale Antikörper (mAB), welche nicht an die in Anspruch 1 definierten CK 19-Sequenzen binden, eignen sich überhaupt nicht für das Verfahren der Erfindung oder ergeben eine Reaktion mit ungenügender klinischer Spezifität. Dies zeigen z.B. Vergleichsversuche gemäß Beispiel 3, die mit den mABs AE1 und b170 durchgeführt wurden. AE1 bindet an ein Epitop auf der Aminosäure-Sequenz 153 bis 219 und ist beschrieben in J.Biol.Chem. 261 (1986) 4646-4654 und in J.Cell.Biol 95 (1982) 580-588. b170 bindet an ein Epitop auf der Aminosäure-Sequenz 336-345, also zwischen den beiden bevorzugten mABs der Erfindung und ist erhältlich nach Lab.lnvest. 55 (1986) 497-504. Die Ergebnisse dieser Versuche zeigt nachstehende Tabelle:

| mAB-Kombination | maligne Krankheiten | | benigne Krankheiten | |
|---|---|---|---|---|
| | untersuchte Seren | positiv | untersuchte Seren | positiv |
| 803¹⁾+804²⁾ | 24 | 11 | 24 | 4 |
| 803 + b170 | 24 | 0 | 24 | 2 |
| b170 + 804 | 24 | 4 | 24 | 1 |
| AE1 + 804 | 24 | 0 | 24 | 0 |

| | | | | |
|---|---|---|---|---|
| ¹⁾ = ECACC 89112803 | | | | |
| ²⁾ = ECACC 89112804 | | | | |

Der Nachweis wird in Körperflüssigkeiten, bevorzugt dem Serum, durchgeführt. Die Bestimmung erfolgt nach an sich bekannten immunologischen Methoden. Für die Durchführung des erfindungsgemäß vorgesehenen immunologischen Bestimmungsverfahrens sind sehr viele Varianten bekannt, die alle hier geeignet sind. So können zwei oder drei oder auch mehr Rezeptoren verwendet werden und die Inkubation mit den einzelnen Rezeptoren kann in verschiedener Reihenfolge in homogener oder heterogener Phase erfolgen. Ausgewertet wird jeweils eine durch Bindung von mindestens zwei Rezeptoren mit dem in der Probelösung nachzuweisenden Fragment erfolgende Signaländerung. Die Verfahrensvarianten sind dem Fachmann bekannt und bedürfen hier keiner näheren Erläuterung. Bevorzugt erfolgt die erfindungsgemäße Bestimmung entweder in homogener Phase z.B. nach dem Prinzip des Agglutinations-Assays, wobei als Rezeptoren beschichtete Partikel wie z.B. Latexpartikel oder Erythrozyten, verwendet werden, die durch Bindung mit spezifisch bindefähigen Rezeptoren und der nachzuweisenden Substanz vernetzen und dadurch agglutinieren oder in heterogener Phase., bevorzugt als Sandwich-Immunoassay. In jedem Falle werden mindestens zwei Rezeptoren R₁ und R₂ eingesetzt, von denen einer einen an Sequen 311 bis 335 von CK 19 bindenden monoklonalen Antikörper enthält, während der andere Rezeptor einen an der Sequenz 346 bis 367 von CK 19 bindenden monoklonalen Antikörper enthält. Geeignet sind auch Antikörper, bei denen eine ausreichende Epitop-Überlappung mit dem in betracht kommenden Antikörpervorliegt. Diese Epitop-Überlappung kann mit Hilfe eines kompetitiven Testsystems leicht nachgewiesen werden. Dazu wird z.B. mit Hilfe eines Enzym-lmmunoassays überprüft, inwieweit ein Antikörper mit einem Antikörper, der mit Verwendung einer der beiden oben angegebenen Bindesequenzen als Immunogen erhalten wurde, um die Bindung an ein definiertes Substrat bzw. ein spezielles Epitop konkurriert. Dazu inkubiert man eine das Fragment der entsprechenden Sequenz enthaltende Lösung mit dem definierten erfindungsgemäßen hergestellten Antikörper in markierter Form und einem Überschuß des in betracht gezogenen Antikörpers. Durch Immobilisierung der gebildeten Komplexe, Trennung der festen von der flüssigen Phase und Nachweis der gebundenen Markierung in einer der beiden Phasen kann dann leicht festgestellt werden, inwieweit der in betracht gezogene monoklonale Antikörper den definierten Antikörper aus der Bindung verdrängen kann. Ist eine Verdrängung von mindestens 50 % bei 10⁵ fachem Überschuß gegeben, so liegt eine Epitop-Überlappung vor und der entsprechende Antikörper ist zum Einsatz für das erfindungsgemäße Verfahren geeignet.

Für die Erfindung geeignete mABs erhältman nach den dem Fachmann hierfür bekannten Methoden unter Verwendung von CK 19 Fragmenten welche die oben definierten geeigneten Sequenzen enthalten oder daraus bestehen. Auch komplettes CK 19 kann verwendet werden.

Bei Inkubation der Körperflüssigkeit mit den beiden Rezeptoren bilden sich nun Komplexe aus R₁, Cytokeratin 19-Fragment und R₂. Die Rezeptoren werden so ausgesucht, daß nur Komplexe, in denen sowohl R₁ als auch R₂ mit dem Cytokeratin 19-Fragment verbunden sind, eine Signaländerung ergeben, so daß auf diese Weise nur solche Fragmente erfaßt werden, die mit den beiden spezifischen Antikörpern bindefähig sind.

Bevorzugt erfolgt die erfindungsgemäße Bestimmung als Sandwich-lmmunoassay. Dazu wird Rezeptor R₁ immobilisiert oder immobilisierbar gemacht und mit der Probelösung umgesetzt. Anschließend wird Rezeptor R₂ zugegeben. Es bilden sich Komplexe aus dem immobilisierten Rezeptor R₁, dem nachzuweisenden CK19-Fragment und Rezeptor R₂. Nur Komplexe, die an die Festphase gebunden sind und eine Markierung tragen, gehen in die Auswertung ein.

In dieser Ausführungsform vermittelt Rezeptor R₁ die Bindung an die feste Phase. Dazu kann Rezeptor R₁ entweder direkt oder über einen Spacer an eine feste Phase gebunden sein oder aber immobilisierbar sein. In einer bevorzugten Ausführungsform ist Rezeptor R₁ ein Konjugat aus einem monoklonalen Antikörper mit der oben angegebenen Spezifität und einer spezifisch bindefähigen Substanz. Der mit der spezifisch bindefähigen Substanz bindefähige Partner ist an eine Festphase gebunden. Als spezifisch bindefähige Paare können beispielsweise Antigen-Antikörper; Hapten-Antikörper; Biotin-Antibiotin-Antikörper; Biotin-Avidin; Biotin-Streptavidin; Protein-A-Immun-γ-Globulin genannt werden. Besonders bevorzugt wird in dieser Ausführungsform als R₁ ein Konjugat des oben angegebenen monoklonalen Antikörpers mit Biotin und als Festphase eine Matrix verwendet, die an ihrer Oberfläche Streptavidin trägt. Durch Bindung des Biotins mit dem Streptavidin erfolgt dann die Immobilisierung des monoklonalen Antikörpers. Bevorzugt ist auch eine Ausführungsform, bei der an der Oberfläche der festen Phase Antikörper gegen den Fc-Teil der für Rezeptor R₁ verwendeten monoklonalen Antikörper oder Protein A-Moleküle gebunden werden, wobei dann durch Bindung der Fc-Teile der monoklonalen Antikörper von R₁ die Immobilisierung erfolgt.

In einer weiteren bevorzugten Ausführungsform sind an eine Matrix Biotinmoleküle gebunden und als Rezeptor R₁ wird ein Konjugat aus Biotin und dem monoklonalen Antikörper verwendet. Die Immobilisierung kann dann nach Durchführung der immunologischen Reaktion durch Zugabe von Streptavidin erfolgen.

Als feste Phase sind die in immunologischen Verfahren üblicherweise verwendeten Materialien geeignet. Beispielsweise können Polymermaterialien sowie Glas eingesetzt werden. Als besonders geeignet haben sich Polystyrol, Polymethacrylat, Teflon, Polyamid, Copolymere aus Styrol und Acrylnitril, Glas- und Celluloseprodukte erwiesen. Die Matrix kann in beliebiger Form vorliegen, z.B. als Röhrchen, Mikrotiterplatte, Kugel, Film, Pulver, Körnchen oder Faservlies. Geeignet sind beispielsweise nach einem in der DE-A 36 40 412 beschriebenen Verfahren erhaltene Festphasen.

Der Rezeptor R₂ enthält in dieser Ausführungsform jeweils den anderen monoklonalen Antikörper, der erfindungsgemäß notwendig ist. Die Markierung des Rezeptors R₂ erfolgt nach bekannten Methoden. Als Markierung sind radioaktive Substanzen, NMR-Signale liefernde Substanzen, Enzyme und fluoreszierende Substanzen geeignet. Der Nachweis der Markierung erfolgt dabei nach bekannten Methoden. Bevorzugt wird als Markierung ein Enzym eingesetzt. Als Enzyme geeignet sind insbesondere Peroxidase, alkalische Phosphatase und β-Galactosidase. Der Nachweis des Enzyms erfolgt durch Zugabe eines Substrats und Messung der gebildeten Farbe.

In derweiteren bevorzugten Ausführungsform des Agglutinationsassays ist Rezeptor R₁ ein mit einem der beiden definierten Antikörper beschichtetes Partikel, während der andere Rezeptor ein mit dem anderen Antikörper beschichtetes Partikel ist. Durch Bindung der Partikel an die nachzuweisende Substanz kommt es zur Agglutination, die über die Trübungsänderung nachgewiesen werden kann.

Mit dem erfindungsgemäßen Verfahren kann das Vorhandensein von bestimmten Fragmenten von Cytokeratin 19 nachgewiesen werden, die zwei Epitope enthalten, die mit den beiden eingesetzten Antikörpern bindefähig sind. Derartige Fragmente entstehen überwiegend in Tumorgewebe.

Ein weiterer Gegenstand der Erfindung ist ein Reagenz zum Nachweis von malignen Erkrankungen, das dadurch gekennzeichnet ist, daß es mindestens zwei Rezeptoren R₁ und R₂ enthält, wobei einer der beiden Rezeptoren einen monoklonalen Antikörper enthält, welcher an die Sequenz 311 bis 335 von Cytokeratin 19 bindet und der andere Rezeptor einen an die Sequenz 346 bis 367 von Cytokeratin 19 bindenden monoklonalen Antikörper oder einen in äquivalenter Weise bindefähigen Antikörper oder dessen Derivate enthält. Bevorzugt enthält das Reagenz den von der Zellinie ECACC 89112803 produzierten und den von der Zellinie ECACC 89112804 produzierten mAB.

Aufgrund ihrer Reaktivität mit den oben erwähnten Epitopen von Cytokeratin 19 kann jeder der beiden mAB zur in vivo-Differenzierung zwischen Epitop-positiven und Epitop-negativen Geweben eingesetzt werden. Dazu wird der Antikörper oder Fab- bzw. (Fab')2-Fragmente desselben an einen für diese Zwecke geeigneten Label gebunden und über geeignete Transportmedien, z.B. nach Injektion über die Blutbahn, an Epitop-positive Gewebe (z.B. Tumoren, Metastasen) gebracht und dort gebunden. Mit bildgebenden Verfahren kann dann der gebundene mAB dargestellt werden. Beispiele für bildgebende Labels sind die Isotope Tc-99, J-131, J-125, In-111 für die radiographische Darstellung und Fe, Cu, Mn, Gd oder F für die kernresonanzmagnetische Darstellung.

Die Erfindung wird durch die folgenden Beispiele erläutert:

### Beispiel 1

Monoklonaler Antikörper BM 19 ECACC 89112804.

### Immunogen

Als Immunogen diente Zytoskelett von MCF-7-Zellen. Das Prinzip der Herstellung dieser Immunogene ist u.a. beschrieben in Meth. in Enzymol. 134, 355 ff. (1986) und Exp. Cell Res. 173, 17 ff. (1987). Zusammengefaßt: aus ca. 10¹⁰ MCF-7-Zellen wurde ein Extrakt mit Detergenz-Puffer (1% Triton) hergestellt Nach Zentrifugation der Homogenate bei 2500 g wurden die Rückstände mit Hochsalzpuffer extrahiert. Der unlösliche Anteil nach dieser Extraktion entsprach der CK-Fraktion. Dieser Anteil wurde als Immunogen verwendet.

### Immunisierung

Balb/c-Mäuse, 6 bis 8 Wochen alt, wurden mit 70 µg der CK 19-Antigen enthaltenden CK-Fraktion in komplettem Freundschem Adjuvans intraperitoneal immunisiert. In dreimonatigem Rhythmus wurden 3 weitere Immunisierungen mit jeweils 70 µg Antigen in inkomplettem Freundschem Adjuvans durchgeführt.

### Fusion und Klonierung

Milzzellen einer immunisierten Maus wurden mit X63-Ag8-653 Myelomzellen (ATCC-CRL 8375) im Verhältnis 1:1 nach dem Standardverfahren gemäß J. of Immunol. Meth., Vol. 39, 285-308 fusioniert.

Während der Subklonierung wurden die CK-spezifischen Klone durch ihre positive Reaktion in derlmmunfluoreszenzmikroskopie herausselektioniert. Für die Immunfluoreszenzmikroskopie wurden Kulturzellen (MCF-7) wie auch Humangewebe (Leber) verwendet.

### Induktion und Ascites

2 bis 5 x 10⁶ Hybridzellen wurden intraperitoneal in mit Pristan vorbehandelte Mäuse gespritzt. Nach 15 bis 20 Tagen konnte Ascites mit einer Antikörperkonzentration von 5 bis 10 mg/ml gewonnen werden.

### Spezifität des monoklonalen Antikörpers BM 19

Der Antikörper hat die Subklasse IgG2b. Er reagiert bei Blot-Analysen mit CK aus verschiedenen Geweben (z.B. Epidermis, Myometrium) und Kulturzellen (z.B. MCF-7, RT 112, A431) ausschließlich mit CK 19.

### Beispiel 2

Monoklonaler Antikörper Ks 19.1 ECACC 89112803 (IgG2a)

### Immunogen

Als Immunogen dienten lebende Zellen der humanen Zelllinie MCF-7.

### Immunisierung

Balb/c-Mäuse wurden fünfmal mit ca. 10⁷ lebenden Zellen i.p. immunisiert.

### Fusion und Klonierung

Es wurde eine Elektrofusion durchgeführt (Eur. J. Clin. Oncol. 21, 733 ff (1985). Als Fusionspartner diente die Plasmacytomlinie X63-Ag8-653.

### Induktion von Ascites

2 bis 5 x 10⁶ Hybridzellen wurden i.p. in mit Pristan vorbehandelte Mäuse injiziert. Nach 10 bis 15 Tagen konnte Ascites mit einer Antikörperkonzentration von 10 bis 15 mg/ml gewonnen werden.

### Beispiel 3

### Testdurchführung für CK 19

Zur Bestimmung des CK 19-Fragments in Körperflüssigkeiten wurde ein Sandwich-Enzym-lmmunoassay durchgeführt. Dabei wurde der monoklonale Antikörper Ks 19.1 (3 µg/ml) als Biotinkonjugat in einem Volumen von 100 µl PBS während einer Stunde bei Raumtemperatur an die streptavidinbeschichtete Mikrotiterplatten-kavität gebunden. Nach viermaligem Waschen mit 0,05 % Tween®/PBS erfolgte die Inkubation mit der Serumprobe (2 µl Serum auf 100 µl PBS) während 90 Minuten bei Raumtemperatur. Danach wird erneut viermal gewaschen mit 0,05 % Tween®/PBS. Anschließend wurde inkubiert mit dem monoklonalen Antikörper BM 19, gekoppelt an Peroxidase (Endkonzentration 250 mU/ml) während 90 Minuten bei Raumtemperur. Nach erneutem viermaligem Waschen mit 0,05 % Tween®/PBS wurde mit der Enzymsubstratlösung ABTS® (100 mmol/l Phosphat-Citrat-Puffer pH 5,0, 1,47 mmol/l Natriumperborat, 9,1 mmol/l ABTS®) bei Raumtemperatur inkubiert und nach 30 Minuten die Extinktion bei 405 nm als Maß für die Analytkonzentration gemessen.

Die Untersuchungen wurden in Seren von Patienten mit verschiedenen Erkrankungen durchgeführt. Die Ergebnisse sind der folgenden Tabelle zu entnehmen.

**Tabelle**

| Test "Cytokeratin 19" | | | |
|---|---|---|---|
| Krankheit | | untersuchte Seren | pos. Seren |
| 1. | Normalseren | 19 | 0 |
| 2. | benigne Krankheiten | | |
| | Schwangere | 20 | 2 |
| | Niereninsuffizienz | 5 | 1 |
| | Pankreatitis | 2 | 0 |
| | Leberzirrhose | 5 | 3 |
| | Morbus Crohn | 5 | 0 |
| | Hepatitis | 5 | 3 |
| | Mastopathie | 1 | 0 |
| | Uterus myo. | 2 | 0 |
| | Autoimmunerkr. | 5 | 1 |
| | prim. bil. | | |
| | Zirrhose | 5 | 1 |
| 3. | maligne Krankheiten | | |
| | Mamma-Ca | 37 | 20 |
| | Ovar-Ca | 8 | 3 |
| | Colon-Ca | 7 | 7 |
| | Pankreas-Ca | 5 | 1 |
| | Magen-Ca | 5 | 3 |
| | Bronchial-Ca | 9 | 4 |
| | Collum-Ca | 5 | 3 |
| | prim. Lebercell-Ca | 5 | 4 |
| | HNO-Ca | 3 | 0 |
| | Gallenwegs-Ca | 4 | 4 |
| | Plasmocytom | 5 | 0 |
| | metast. Hodentumor | 2 | 0 |
| | Prostata-Ca | 2 | 1 |
| | Neuroblastom | 1 | 0 |
| | Colitis ulc. | 1 | 0 |

### Beispiel 4

Es wurde die Epitop-Überlappung eines Antikörpers mit dem monoklonalen Antikörper, ECACC 89112804 bestimmt. Der Nachweis erfolgt im Rahmen eines kompetitiven Enzym-Immunoassays. Dazu wird der monoklonale Antikörper, z.B. ECACC 89112803 (3 µg/ml) als Biotin-Konjugat in einem Volumen von 100 µl PBS (8 g/l NaCI, 0,2 g/l KCI, 1,44 g/l NaH₂PO₄x2H₂O, 0,2 g/l KH₂PO₄) während 1 Stunde bei Raumtemperatur an die Streptavidin beschichtete Mikrotiterplatten-Kavität gebunden, Nach viermaligem Waschen mit 0,05 % Tween® 20/PBS erfolgte die Inkubation mit einer hochtitrigen Serumprobe (2 µl Serum auf 100 µl PBS) während 90 Minuten bei Raumtemperatur. Danach wurde erneut viermal gewaschen mit 0,05 % Tween® 20/PBS. Anschließend wurde während 90 Minuten bei Raumtemperatur simultan inkubiert mit dem monoklonalen Antikörper, z.B. ECACC 89112804, markiert mit Peroxidase (Endkonzentration 250 mU/ml) und dem zu beurteilenden Antikörper. Nach erneutem viermaligem Waschen mit 0,05 % Tween® 20/PBS wurde mit der Enzymsubstratlösung ABTS® (ABTS® = 2,2'-Azino-di-[3-ethyl-benzthiazolin-sulfonsäure(6)]-diammoniumsalz) bei Raumtemperatur inkubiert und nach 30 Minuten die Extinktion bei 405 nm als Maß für die Analytkonzentration gemessen. Dieser Wert wurde verglichen mit der Extinktion, die erhalten wurde bei Inkubation mit dem monoklonalen Antikörper, ECACC 89112804 allein. Wenn bis zu einem 10⁵ fachen Überschuß an zu beurteilendem Antikörper gegenüber dem monoklonalen Antikörper ECACC 89112804 Enzymkonjugat (250 mU/l) mindestens 50 % Kompetition zu erkennen sind, liegt eine Epitopüberlappung vor.

### Beispiel 5

a) Markierung von Antikörper mit J-125
   Die in den Beispielen 1 bis 4 beschriebenen Antikörper oder Fragmente derselben werden mit 1 mCi J-125 nach der Chloramin-T-Methodejodiert (Biochem.J. 89, 114-123 (1963)). Anschließend wird das nicht umgesetzte Jod über eine Sephadex® G-50-Säule (Pharmacia) abgetrennt und die Immunreaktivität der monoklonale Antikörper im ELISA überprüft.
b) Tumorlokalisation mit jodmarkiertem monoklonalem Antikörper
   Nackte Mäuse (Balb/c nu/nu) werden subkutan mit 3-5 x 10¹⁰ HeLa-Zellen (Typ 2) inokuliert. Nach ca. 8 Wochen werden diejenigen Mäuse, welche einen soliden Tumor gebildet haben, mit 200 µl Kl (Kaliumjodid) (1 mg/ml) in PBS (phosphate-buffered saline) gespritzt. 24 h nach der KI-Injektion werden ca. 50 µg des markierten monoklonalen Antikörpers bzw. 70 µg des entsprechenden Antikörper-Fragments injiziert. Die Verteilung der Radioaktivität wird über 20 Tage mit Hilfe eines autoradiographischen Gerätes (z.B. von General Electric) beobachtet. Eine Akkumulation der Radioaktivität im Bereich des soliden Tumors kann dabei aufgrund der spezifischen Bindung mit beiden monoklonalen Antikörper beobachtet werden.

## Patentansprüche

1. Verfahren zum Nachweis von malignen Erkrankungen,
**dadurch gekennzeichnet, daß** man die Probe einer Körperflüssigkeit mit mindestens zwei Rezeptoren R₁ und R₂ inkubiert, wobei durch Bindung mindestens der Rezeptoren R₁ und R₂ mit der in der Probelösung nachzuweisenden Substanz eine Signaländerung erzeugt wird und wobei einer der beiden Rezeptoren einen monoklonalen Antikörper enthält, welcher an die Aminosäuresequenz 311 bis 335 von Cytokeratin 19 bindet und der andere Rezeptor einen monoklonalen Antikörper, welcher an die Aminosäuresequenz 346 bis 367 von Cytokeratin 19 bindet, enthält und die durch die Bindung verursachte Signaländerung in der Probe bestimmt.

2. Verfahren nach Anspruch 1, **dadurch gekennzeichnet, daß** man als Rezeptor R₁ einen Rezeptor verwendet, der die Bindung an eine feste Phase vermittelt und als Rezeptor R₂ einen markierten Rezeptor verwendet und die Markierung nach Trennung der festen von derflüssigen Phase in einer der beiden Phasen bestimmt.

3. Verfahren nach Anspruch 2, **dadurch gekennzeichnet, daß** eine Festphase, die mit Streptavidin beschichtet ist und als Rezeptor R₁ ein Konjugat eines der beiden in Anspruch 1 definierten Antikörper mit Biotin verwendet wird, wobei die Bindung des monoklonalen Antikörpers an die Festphase durch die Bindung von Biotin an Streptavidin erfolgt.

4. Verfahren nach einem der Ansprüche 2 oder 3,
**dadurch gekennzeichnet, daß** als Rezeptor R₂ ein monoklonaler Antikörper wie in Anspruch 1 definiert verwendet wird, der radioaktiv, mit einer fluoreszierenden oder NMR-Signale liefernden Substanz oder einem Enzym markiert ist.

5. Verfahren nach einem der vorhergehenden Ansprüche,
**dadurch gekennzeichnet, daß** monoklonale Antikörper verwendet werden, von denen einer an Sequenz 311 bis 335, der andere an Sequenz 346 bis 359 von Cytokeratin 19 bindet.

6. Verfahren nach Anspruch 5,
**dadurch gekennzeichnet, daß** als an Sequenz 311 bis 335 bindender Antikörper der von der Zellinie ECACC 89112803 gebildete Antikörper oder ein in äquivalenter Weise bindefähiger Antikörper verwendet wird.

7. Verfahren nach Anspruch 5,
**dadurch gekennzeichnet, daß** als an Sequenz 346 bis 359 bindender Antikörper der von der Zellinie ECACC 89112804 gebildete Antikörper oder ein in äquivalenter Weise bindefähiger Antikörper verwendet wird.

8. Reagenz zum Nachweis von malignen Erkrankungen,
**dadurch gekennzeichnet, daß** es mindestens zwei Rezeptoren R₁ und R₂ enthält, wobei einer der beiden Rezeptoren einen monoklonalen Antikörper enthält, welcher an die Sequenz 311 bis 335 von Cytokeratin 19 bindet und der andere Rezeptor einen an die Sequenz 346 bis 367 von Cytokeratin 19 bindenden monoklonalen Antikörper enthält.

9. Reagenz nach Anspruch 8,
**dadurch gekennzeichnet, daß** es als Rezeptor R₂ einen monoklonalen Antikörper enthält, welcher an die Aminosäuresequenz 311 bis 335 oder 346 bis 367 von Cytokeratin 19 bindet.

10. Reagenz nach Anspruch 8 oder 9,
**dadurch gekennzeichnet, daß** es eine Festphase, die mit Streptavidin beschichtet ist und als Rezeptor R₁ ein Konjugat eines monoklonalen Antikörpers, welcher an die Aminosäuresequenz 311 bis 335 oder 346 bis 367 von Cytokeratin 19 bindet und von dem Antikörper gemäß Anspruch 9 verschieden ist, mit Biotin enthält.

11. Reagenz nach Anspruch 9 oder 10,
**dadurch gekennzeichnet, daß** es den von der Zellinie ECACC 89112803 gebildeten Antikörper oder einen in äquivalenter Weise bindefähigen Antikörper enthält.

12. Reagenz nach einem der Ansprüche 9 oder 10,
**dadurch gekennzeichnet, daß** es einen monoklonalen Antikörper enthält, welcher an die Aminosäure-sequenz 346 bis 359 von Cytokeratin 19 bindet.

13. Reagenz nach Anspruch 12,
**dadurch gekennzeichnet, daß** es den von der Zellinie ECACC 89112804 gebildeten Antikörper oder einen in äquivalenter Weise bindefähigen Antikörper enthält.

## Claims

1. A method of detecting malignant diseases,
**characterized by** incubating the sample of a body fluid with at least two receptors R₁ and R₂, a signal change being produced by binding at least said receptors R₁ and R₂ to the substance to be detected in the sample solution and one of the two receptors containing a monoclonal antibody which binds to the amino acid sequence 311 to 335 of cytokeratin 19 and the other receptor containing a monoclonal antibody which binds to the amino acid sequence 346 to 367 of cytokeratin 19, and by determining the signal change caused by the binding in the sample.

2. The method according to claim 1, **characterized by** using as receptor R₁ a receptor which mediates binding to a solid phase, and by using as receptor R₂ a labeled receptor and by determining the labeling after separating the solid phase from the liquid phase in one of the two phases.

3. The method according to claim 2, **characterized by** using a solid phase which is coated with streptavidine and a conjugate of one of the two antibodies defined in claim 1 with biotin as receptor R₁, the monoclonal antibody being bound to the solid phase by binding biotin to streptavidine.

4. The method according to any of claims 2 or 3,
**characterized in that** a monoclonal antibody as defined in claim 1 is used as receptor R₂, which is labeled radioactively with a fluorescent or NMR signals-supplying substance or an enzyme.

5. The method according to any of the preceding claims,
**characterized in that** monoclonal antibodies are used one of which binds to sequence 311 to 335 and the other binds to sequence 346 to 359 of cytokeratin 19.

6. The method according to claim 5,
**characterized in that** the antibody formed by cell line ECACC 89112803 or an antibody with equivalent binding capacity is used as the antibody binding to sequence 311 to 335.

7. The method according to claim 5,
**characterized in that** the antibody formed by cell line ECACC 89112804 or an antibody with equivalent binding capacity is used as the antibody binding to sequence 346 to 359.

8. A reagent for detecting malignant diseases,
**characterized in that** it contains at least two receptors R₁ and R₂, one of the two receptors containing a monoclonal antibody which binds to the sequence 311 to 335 of cytokeratin 19 and the other receptor containing a monoclonal antibody binding to sequence 346 to 367 of cytokeratin 19.

9. The reagent according to claim 8,
**characterized in that** it contains a monoclonal antibody as receptor R₂, which binds to the amino acid sequence 311 to 335 or 346 to 367 of cytokeratin 19.

10. The reagent according to claim 8 or 9,
**characterized in that** it contains a solid phase coated with streptavidine and as receptor R₁ a conjugate of a monoclonal antibody, which binds to the amino acid sequence 311 to 335 or 346 to 367 of cytokeratin 19 and differs from the antibody according to claim 9, with biotin.

11. The reagent according to claim 9 or 10,
**characterized in that** it contains the antibody formed by the cell line ECACC 89112803 or an antibody with equivalent binding capacity.

12. The reagent according to any of claims 9 or 10,
**characterized in that** it contains a monoclonal antibody which binds to the amino acid sequence 346 to 359 of cytokeratin 19.

13. The reagent according to claim 12,
**characterized in that** it contains the antibody formed by cell line ECACC 89112804 or an antibody with equivalent binding capacity.

## Revendications

1. Procédé de mise en évidence de maladies malignes, **caractérisé en ce que** l'on incube l'échantillon d'un liquide biologique avec au moins deux récepteurs R₁ et R₂, une modification de signal apparaissant par liaison d'au moins les récepteurs R₁ et R₂ avec la substance qui doit être mise en évidence dans la solution échantillon et l'un des deux récepteurs contenant un anticorps monoclonal qui se lie à la séquence d'acides aminés 311 à 335 de la cytokératine 19 et l'autre récepteur contenant un anticorps monoclonal qui se lie à la séquence d'acides aminés 346 à 367 de la cytokératine 19, et l'on détermine dans l'échantillon la modification de signal provoquée par la liaison.

2. Procédé selon la revendication 1, **caractérisé en ce que** l'on utilise comme récepteur R₁ un récepteur qui permet la liaison à une phase solide et comme récepteur R₂ un récepteur marqué et l'on détermine le marqueur dans l'une des deux phases après séparation de la phase solide d'avec la phase liquide.

3. Procédé selon la revendication 2, **caractérisé en ce que** l'on utilise une phase solide qui est recouverte de streptavidine et comme récepteur R₁ un conjugué de l'un des deux anticorps définis dans la revendication 1 et de biotine, la liaison de l'anticorps monoclonal à la phase solide se faisant par la liaison de la biotine à la streptavidine.

4. Procédé selon l'une des revendications 2 ou 3, **caractérisé en ce que** l'on utilise comme récepteur R2 un anticorps monoclonal tel que défini dans la revendication 1 qui est marqué de manière radioactive, avec une substance fluorescente ou produisant des signaux RMN ou avec une enzyme.

5. Procédé selon l'une des revendications précédentes, **caractérisé en ce que** l'on utilise des anticorps monoclonaux dont l'un se lie à la séquence 311 à 335 et l'autre se lie à la séquence 346 à 359 de la cytokératine 19.

6. Procédé selon la revendication 5, **caractérisé en ce que** l'on utilise comme anticorps qui se lie à la séquence 311 à 335 l'anticorps formé par la lignée cellulaire ECACC 89112803 ou un anticorps capable de se lier de manière équivalente.

7. Procédé selon la revendication 5, **caractérisé en ce que** l'on utilise comme anticorps qui se lie à la séquence 346 à 359 l'anticorps formé par la lignée cellulaire ECACC 89112804 ou un anticorps capable de se lier de manière équivalente.

8. Réactif de mise en évidence de maladies malignes, **caractérisé en ce qu'**il contient au moins deux récepteurs R₁ et R₂, l'un des deux récepteurs contenant un anticorps monoclonal qui se lie à la séquence 311 à 335 de la cytokératine 19 et l'autre récepteur contenant un anticorps monoclonal qui se lie à la séquence 346 à 367 de la cytokératine 19.

9. Réactif selon la revendication 8, **caractérisé en ce qu'**il contient comme récepteur R₂ un anticorps monoclonal qui se lie à la séquence d'acides aminés 311 à 335 ou 346 à 367 de la cytokératine 19.

10. Réactif selon la revendication 8 ou 9, **caractérisé en ce qu'**il contient une phase solide qui est recouverte de streptavidine et comme récepteur R₁ un conjugué d'un anticorps monoclonal qui se lie à la séquence d'acides aminés 311 à 335 ou 346 à 367 de la cytokératine 19 et qui est différent de l'anticorps selon la revendication 9 et de biotine.

11. Réactif selon la revendication 9 ou 10, **caractérisé en ce qu'**il contient l'anticorps formé par la lignée cellulaire ECACC 89112803 ou un anticorps capable de se lier de manière équivalente.

12. Réactif selon l'une des revendications 9 ou 10, **caractérisé en ce qu'**il contient un anticorps monoclonal qui se lie à la séquence d'acides aminés 346 à 359 de la cytokératine 19.

13. Réactif selon la revendication 12, **caractérisé en ce qu'**il contient l'anticorps formé par la lignée cellulaire ECACC 89112804 ou un anticorps capable de se lier de manière équivalente.
